# EUROPEAN PATENT SPECIFICATION

(11) **EP 1 516 591 B1**
(45) Date of publication and mention of the grant of the patent: **05.11.2008**
(21) Application number: 04021825.7
(22) Date of filing: 14.09.2004
(51) Int. Cl.: A61B 17/22

(54) **Basket-type clamping forceps**
Drahtkorbzange
Pince en panier de fils

(30) Priority: 16.09.2003 JP 2003323386
(43) Date of publication of application: 23.03.2005
(73) Proprietor: Olympus Corporation, Shibuya-ku, Tokyo (JP)
(72) Inventor: Komiya, Takaaki, Akiruno-shi, Tokyo (JP)
(74) Representative: von Hellfeld, Axel

(56) References cited:
- US-A- 4 633 871
- US-A- 4 865 017
- US-A- 5 059 199
- US-A- 6 093 196
- PATENT ABSTRACTS OF JAPAN vol. 2000, no. 04, 31 August 2000 (2000-08-31) & JP 2000 005186 A (OLYMPUS OPTICAL CO LTD), 11 January 2000 (2000-01-11)

## Description

### TECHNICAL FIELD

The present invention relates to a basket-type clamping forceps.

### BACKGROUND ART

Conventionally, a basket-type clamping forceps is used for a handling apparatus for clamping an irregular member such as a calculus which is generated in a coelom such as a biliary so as to be inserted in the coelom oral-endoscopically. A proposal has been made for such a basket-type clamping forceps in which a plurality of wire assemblies are provided with a plurality of flexible wires, a tip of the wire assembly and a rear end of the wire assembly is bundled, a plurality of bending points which are provided with a convex bending points under a convex bending condition in a middle of the flexible wire in a direction which is directed to a rear end of the concave bending point under concave bending condition in a direction in which the read end is separated from a virtual line between the tip and the rear end in a linear line condition; thus, a structure is that the basket-type clamping forceps has a basket section which is formed to be like a basket entirely (See FIGS. 5 and 6 in Japanese Unexamined Patent Application, First Publication No. 3,075,355).

In such a basket-type clamping forceps, a fall-off of a member such as a calculus, etc. which is clamped in the basket section is prevented. Therefore, an opening angle between the flexible wires in a wire assembly under an opening condition and an opening angle between the flexible wires in a neighboring wire assemblies are formed in an approximately the same angle when it is viewed from a tip of the basket section. By doing this, a basket section is contracted by clamping the calculus, etc. in the basket section. Therefore, it is possible to clamp the calculus, etc. in the flexible wire.

However, in the above conventional, a concave bending point which tends to contract toward a center axis of the basket section for the above virtual line is formed near the tip of the basket section so as to contact a wall of a lumen with the convex bending point which is the nearest to the tip or near the rear end. Therefore, when the basket section receives a force which is disposed to an inner section of a radial direction of a lumen, a portion of the concave bending section near the tip is bent toward the center axis. Accordingly, intervals between the wire assemblies near the tip are dense. On the other hand, an interval between the flexible wires in a wire assembly hardly changes. Therefore, an interval between the flexible wires are not equal. Therefore, there has been a possibility in that the calculus, etc. which is clamped inside of the base end for the basket section may fall off from the tip.

Other examples of basket-type grasping tools or clamping forceps are known from published JP 2000-005186, US 5,059,199 and US 6,093,196.

### DISCLOSURE OF INVENTION

The present invention was made inconsideration of the above problem. An object of the present invention is to provide a basket-type clamping forceps which can clamp a calculus, etc. by forming an interval between the flexible wires equally in a narrow coelom.

The present invention provides a basket-type clamping forceps as defined in claim 1 for solving the above problem. Preferred features of the invention are recited in the dependent claims.

A basket-type clamping forceps according to the present invention comprises a flexible operating tube which can be inserted into a forceps' channel in an endoscope, an operating wire section which is inserted in the flexible operating tube so as to be freely move forwardly and backwardly, a handling section of which base end is connected to a tip of the operating wire section so as to freely expand and contract such that the handling section is provided with a plurality of wire assemblies which are formed by a plurality of flexible wires of which tip and base end are bundled respectively, a plurality of bending points and a branch point which branches the flexible wires with an interval from each other are formed in a middle of the base end of the wire assemblies and the tip of the wire assemblies, the wire assemblies are bent under a concave bending condition with reference to an axial line which is disposed between the tip of the handling section and the base end of the handling section in a first bending point and the wire assemblies are bent with reference to the axial line under a convex bending condition in a second bending point under condition that the first bending point is disposed nearest to the tip of the wire assemblies and the second bending point is disposed the second nearest to the tip of the wire assemblies.

The basket-type clamping forceps is provided with the above structure. Therefore, the second bending point hardly bends toward to the axial line; thus, a movement amount in a direction to the axial line can be restricted in a small amount even if a force is applied toward an inner direction of a radius of a handling section from an inner wall of lumen when the handling section is inserted in a lumen which is an object of a biliary. Therefore, it is possible to restrict that the flexible wires in the neighboring wire assemblies may contact with each other nearer to the tip than the second bending point. Also, the interval between the flexible wires in a wire assembly can hardly change. Therefore, it is possible to restrict an unequal interval between the flexible wires. Also, it is possible to maintain the opening angle between the flexible wires in a wire assembly when it is viewed from a tip of the handling section and the opening angle between the flexible wires in a neighboring wire section in an approximately the same angle.

Also, in the basket-type clamping forceps according to the present invention, it is preferable that a distance between the axial line and the first convex bending point is not longer than 3/4 of a distance between the axial line and the second convex bending point under condition that the first convex bending point is disposed the nearest to the tip of the handing section and second convex bending point is disposed the second nearest to the tip of the handing section.

The present basket-type clamping forceps is provided with the above structure; therefore, it is possible to contact the inner wall of lumen near at least the base end from the second convex bending point when the handling section is inserted in the lumen which is an object of the biliary. Therefore, the first convex bending point does not receive a force in an inner direction of a diameter even if a force is applied to an inside of the radius of the handling section. Here, a moving amount of each of the convex bending point in a convex bending shape is restricted so as to be small in an axial direction. Therefore, it is possible to restrict that the flexible wires in the neighboring wire assemblies may contact with each other nearer to the tip than the first bending point.

Also, the interval between the flexible wires in a wire assembly can hardly change. Therefore, it is possible to restrict an unequal interval between the flexible wires. Also, it is possible to maintain the opening angle between the flexible wires in a wire assembly when it is viewed from a tip of the handling section and the opening angle between the flexible wires in a neighboring wire section in an approximately the same angle.

Also, it is preferable that, a basket-type clamping forceps according to the present invention, a plurality of convex bending points are formed in a middle of the bundling section at the tip of the flexible wire and the branch point continuously.

This basket-type clamping forceps is provided with the above explained structure; thus, it is possible to maintain a part of the flexible wire in the wire assembly between the continuous convex bending points such that the flexible wires should not approach each other even if the handling section contracts. Therefore, it is possible to maintain an interval near the tips of the flexible wires in the lumen which is an object such as biliary, etc. in a larger size.

Also, it is preferable that, a basket-type clamping forceps according to the present invention is the above explained basket-type clamping forceps such that a distance between the convex bending points which are formed in a middle of the bundling section of the tips of the flexible wires and the branch points are formed to be approximately equal.

This basket-type clamping forceps is provided with the above explained structure; thus, it is possible to restrict a bending amount of the flexible wire toward an innermore there even if the basket-type clamping forceps receives a force toward an innermore in a radial direction of the lumen which is an object such as the biliary, etc. and form an interval between the flexible wires which are nearer to the tips than the branch point equal.

Also, the basket-type clamping forceps according to the present invention is the above explained basket-type clamping forceps such that an interval among the flexible wires in the neighboring wire assemblies is formed so as to be greater than an interval between the flexible wires in the wire assemblies in the bending points which is bent under bending condition first from the tip of the handling section.

This basket-type clamping forceps is provided with the above explained structure; therefore, the distance between the tip of the handling section and the base terminal is greater than the distance between the tip and the bundling section when the interval between the wires is formed equal after the bending point under the convex bending condition is disposed near the tip so as to be contracted when the handling section is contracted in the lumen which is an object of the biliary, etc.

Therefore, it is possible to maintain the interval between the tips of the flexible wires more desirably when the handling section is contracted even if a contracting amount of the interval between the flexible wires in each of the neighboring wire assemblies is greater than the contracting amount of the interval between the flexible wires in a wire assembly undesirably.

According to the present invention, it is possible to eliminate the calculus, etc which is clamped from the base end of the handling section toward an innermore there by clamping effectively without losing the calculus, etc. from the tips.

### BRIEF DESCRIPTION OF DRAWINGS

FIG. 1 is a side view including a part of a cross section under condition that the basket-type clamping forceps according to a first embodiment of the present invention is inserted in an endoscope.
FIG. 2 is a cross section of the basket-type clamping forceps according to the first embodiment of the present invention.
FIGS. 3A and 3B are side views for showing a handling section of the basket-type clamping forceps according to a first embodiment of the present invention.
FIGS. 4A and 4B are views for showing X-X cross section and Y-Y cross section of FIG. 2.
FIGS. 5A to 5F are views for showing important sections under condition that the handling sections of the basket-type clamping forceps according to the first embodiment of the present invention and the conventional basket-type clamping forceps are inserted in the lumens respectively.
FIGS. 6A to 6B are views for showing important sections under condition that the handling sections of the basket-type clamping forceps according to the first embodiment of the present invention and the conventional basket-type clamping forceps are inserted in the lumens respectively.
FIGS. 7A and 7B are side views for showing a handling section of the basket-type clamping forceps according to a second embodiment as a comparative example not forming part of the present invention.
FIGS. 8A and 8B are side views for showing a handling section of the basket-type clamping forceps according to a third embodiment of the present invention.
FIG. 9 is a front view for showing a handling section of the basket-type clamping forceps according to the third embodiment of the present invention.
FIGS. 10A and 10B are side views for showing a handling section of the basket-type clamping forceps according to other embodiments of the present invention.
FIGS. 11A and 11B are views for showing cross sections in FIG.S 10A and 10B which correspond to X-X position and Y-Y position in FIG. 2.
FIG. 12 is a side view for showing a handling section of the basket-type clamping forceps according to other embodiments of the present invention.
FIG. 13 is a side view for showing a handling section of the basket-type clamping forceps according to other embodiments of the present invention.
FIG. 14A is a general view for a basket-type clamping forceps according to the above aspect of the present invention. FIG. 14B is a cross section which is viewed in a cross section which is orthogonal to a direction of an arrow shown in FIG. 14A.

### DESCRIPTION OF PREFERRED EMBODIMENTS

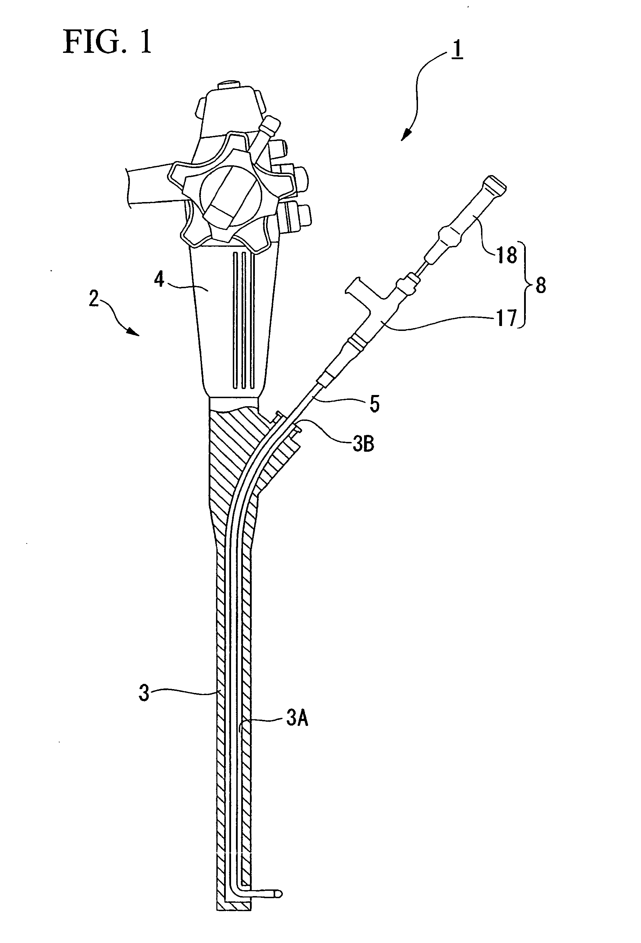

A first embodiment for the basket-type clamping forceps according to the present invention is explained with reference to FIGS. 1 to 4.

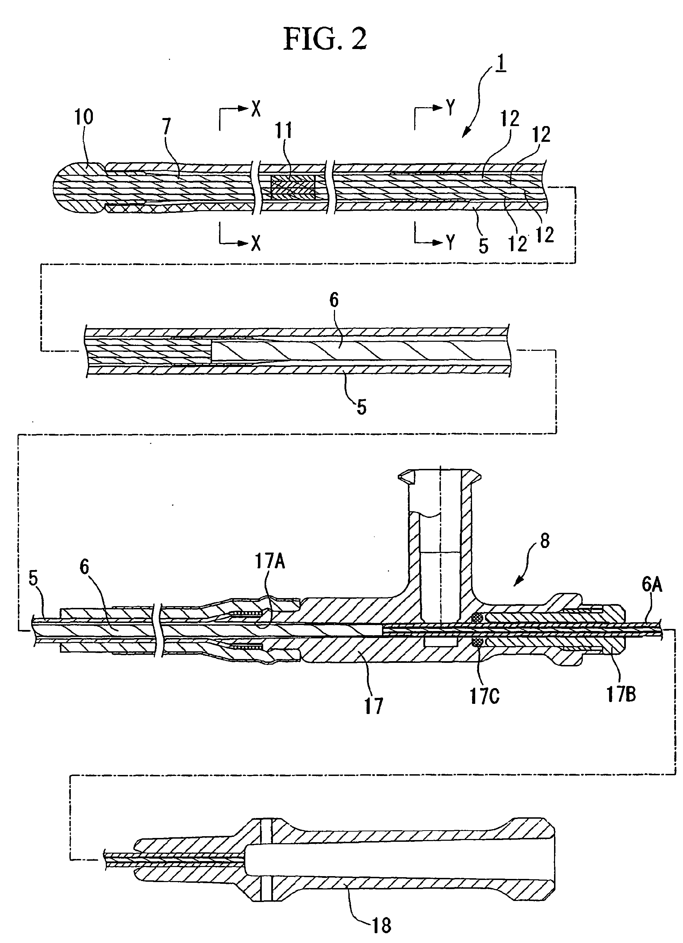

The basket-type clamping forceps according to the present embodiment is used with an endoscope 2 as shown in FIGS. 1 and 2.

A forceps' channel 3A is formed inside of a flexible insertion section 3 in the endoscope 2. A port for forceps 3B is formed on the base end of the forceps' channel 3A. Also, an operating section 4 for endoscope is connected to the base end of the insertion section 3.

The basket-type clamping forceps 1 is provided with an flexible operating tube 5 which can be inserted from the port 3B for forceps, an operating wire section 6 which is inserted in the operating tube 5 so as to freely move forwardly and backwardly, a handling section 7 of which base end is connected to the tip of the operating wire section 6 so as to freely open and contract, and an operating section 8 which is connected to the base end of the operating tube so as to open or contract the handling section 7 by a forward and backward movement.

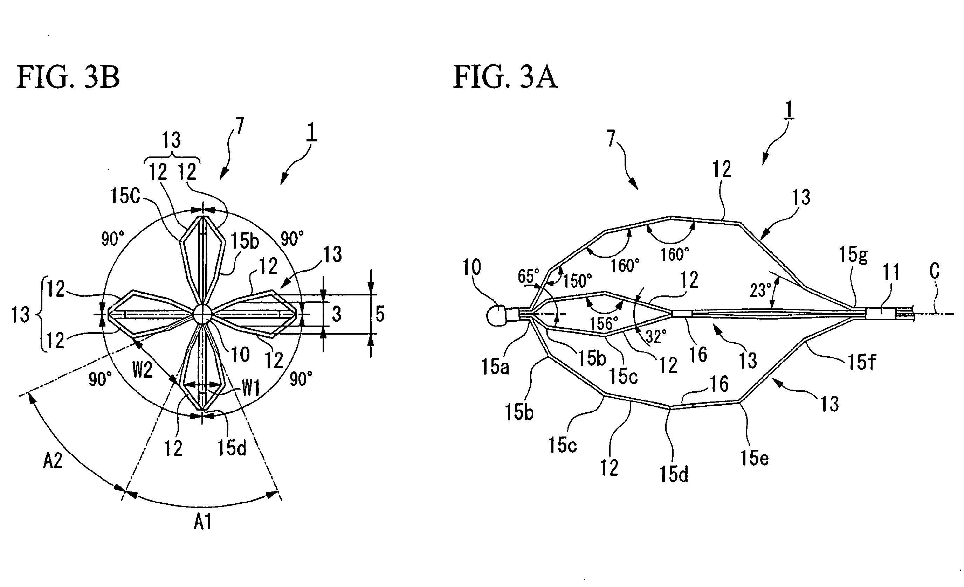

As shown in FIGS. 3A and 3B, the handling section 7 are provided with four wire assemblies 13 which are formed by two flexible wires 12 of which tip and the base end are bundled in the tip bundling section 10 and a base end bundling section 11. Thus, seven bending points 15a to 15g and a branch point 16 which branches the flexible wires 12 by intervals with each other are formed in a corresponding position in an axial direction in a middle between the base ends and the tips of these wire assemblies 13.

As shown in FIG. 3A, the wire assemblies 13 are bent in a first bending point 15a, a sixth bending point 15f, and a seventh bending point 15g are bent under concave bending condition with reference to a central axis C which is an axial line of the handling section 7 which is disposed in a middle of the tip bundling section 10 and the base end bundling section 11 under condition that a bending point which is disposed the nearest to the tip of the wire assembly 13 is a first bending point 15a, a second nearest bending point is a second bending point 15b, a third nearest bending point is a third bending point 15c until a seventh bending point 15g. Also, the second bending point (a first convex bending point) 15b and a portion which covers the third bending point (second convex bending point) 15c to the fifth bending pint 15e are bent under a convex bending condition with reference to the central axis C such that three convex bending points are formed in a section which covers the tip bundling section 10 and the branch point 16.

Each wire assembly 13 is disposed in a three-dimensional relationship in a symmetric manner with reference to the central axis C such that an entire wire assembly should be formed in a basket-like-manner. Also, as shown in FIG. 3B, an angle A2 which is formed by the neighboring flexible wires 12 of the wire assembly 13 and an angle A1 which is formed by the flexible wire 12 which forms a wire assembly 13 are approximately equal when it is views from the tip bundling section 10.

Here, the convex bending condition indicates a condition under which a bending operation is performed in an approaching direction to the central axis C from the tip to the base end with reference t a linear condition. The concave bending condition indicates a condition under which a banding operation is performed in a separating direction in which the base end separates from the central axis C.

Here, a bending operation is performed in the first bending point 15a by 65 ±5 degrees with reference to the central axis C outwardly. A bending operation is performed in the second bending point by 30 ± 5 degrees with reference to a linear condition. Bending operations are performed in the third bending point 15c and the fourth bending point 15d by 20 ±5 degrees with reference to the linear condition toward the base end. Also, a bending operation is performed in the seventh bending point 15g by 23 ±5 degrees with reference to the central axis C outwardly

Each distance between the bending points are formed as follows. That is, a distance between the first bending point 15a and a second bending point 15b (hereinafter called an interval ab) is formed within a range of 7 ±2 mm. An interval between the second bending point 15b and the third bending point 15c (hereinafter called an interval bc), an interval between the third bending point 15c and the fourth bending point d (hereinafter called an interval cd), and an interval between the forth bending point 15d and the fifth bending point 15e (hereinafter called de) are formed within a range of 9 ±2 mm such that the distance between the bending points which are formed between the tip bundling section 10 of the flexible wire 12 and the branch point 16 are formed to be approximately equal.

An interval between the fifth bending point 15e and the sixth bending point 15f (hereinafter called an interval ef) is formed within a range of 12 ±2 mm. Also, an interval between the second bending points 15b of the flexible wires 12 in each of the wire assembly 13 is set at 3 ±2 mm. An interval between the third bending points 15c of the flexible wires 12 in each of the wire assembly 13 is set at 5 ±2 mm.

The interval which is formed by branching the flexible wire 12 between the base end bundling section 11 and the branch point 16 is branched by 32 ±5 degrees toward the third bending point 15c from the branch point 16 so as to simultaneously be bent by 24 ±5 degrees in an approaching direction from a linear condition in the third bending point 15c.

The branch point 16 is disposed so as to neighbor the fourth bending point 15d such that two flexible wires 12 of the wire assembly 13 are fixed by a fixing agent according to a method such as a brazing operation, a soldering operation, and a bonding agent and thermally-contractive tube.

In the wire assembly 13, two flexible wires 12 are disposed in approximately parallel so as to be near with each other from the base end bundling section 11 to this branch point 16. Two flexible wires 12 are disposed so as to expand while separating with each other toward its tip from the branch point 16 to the third bending point 15c. Two flexible wires 12 are bundled so as to neighbor with each other toward the tip from the second bending point 15b. When the handling section 7 is viewed from the tip of the tip, an interval W2 between the neighboring flexible wires 12 in the different wire assemblies 13 is formed so as to be greater than an interval W1 between each of the flexible wires 12 in a common wire assembly 13 in a first bending point 15b which is formed nearest to the tip of the handling section 7 in a similar manner to a feather of an arrow shaft under a convex bending condition as shown in FIG. 3B.

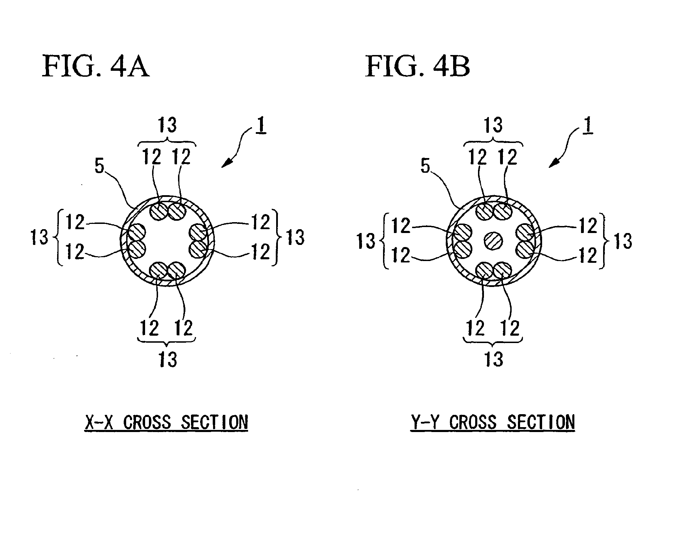

Here, a diameter of each flexible wire 12 is formed equally as shown in FIGS. 4A and 4B.

The operating wire section 8 is provided with a main body 17 which is formed so as to expand in a direction toward the operating wire section 6, a sliding section 18 which is connected to an operating bar member 6A which expands from the base end of the operating wire section so as to be engaged with the main body 17 so as to freely move forwardly and backwardly.

A through-hole 17A through which the operating bar member 6A can pass is formed in an axial direction of the main body. A supporting section 17B in which an operating bar member 6A can slide is disposed on the base end of the main body 17. Thus, an air-tight condition is maintained by an O-ring 17C from thereoutside if the operating bar member 6A moves forwardly and backwardly.

Next, a first operating method and effect by the basket-type clamping forceps according to the present embodiment is explained with reference to a case in which, for example, a biliary calculus is collected.

First, as shown in FIGS. 1 and 2, the handling section 7 is inserted in the coelom via the forceps' channel 3A in the insertion section 3 of the endoscope 2 which is inserted in the coelom under condition that the handling section 7 is contained in the operating tube 5. Consequently, the tip of the operating tube 5 is protruded from the tip of the forceps' channel 3A so as to be inserted in, for example, a gallbladder from a papilla duodeni.

When the tip of the handling section 7 goes behind a position of the calculus, he Sliging section 18 is operated so as to move forwardly and move the operating wire section 6 forwardly; thus, the handling section 7 is disposed so as to protrude from the tip of the operating tube 5. In such a case, the handling section 7 opens in a basket-like-manner until the handling section 7 contacts an inner wall of the gallbladder by a flexibility force which intends to open each of the bending points 15a to 15g which are formed in each of the wire assemblies 13.

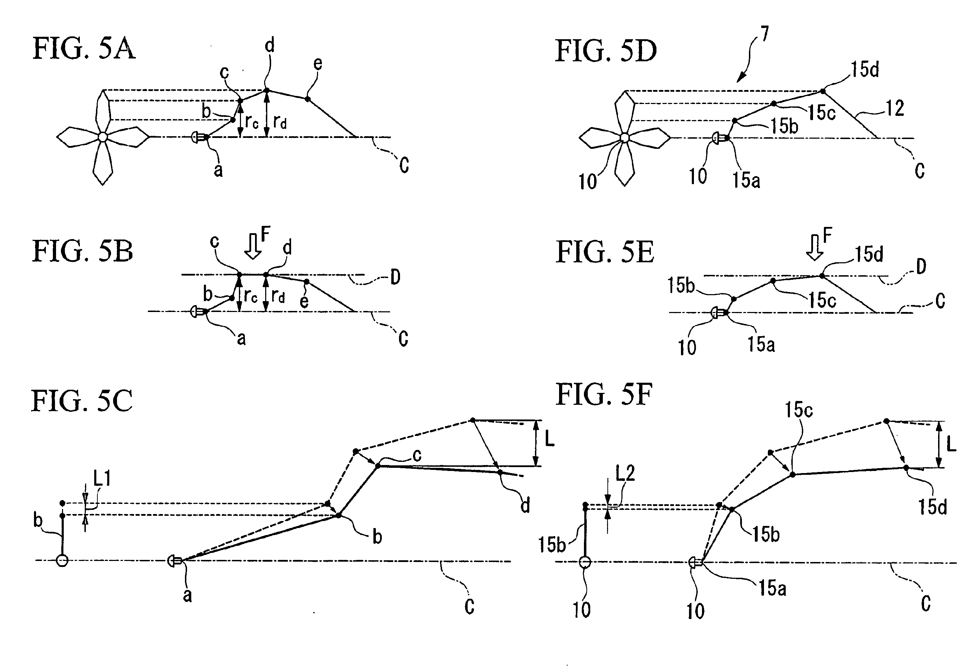

In such a case, as shown in FIG. 5A, if a conventional basket type clamping forceps in which a distance rc between the third bending point c and the central axis such that the second bending point b is a concave bending point and a distance rd between the fourth bending point d and the central axis are approximately equal receives a force F in a direction to an inside of a radial direction as a counter force to the flexibility force when the basket type clamping forceps contacts the inner wall D of the gallbladder at the fourth bending point under convex bending condition as shown in FIG. 5B, the rc and the rd are equal; thus, the force F reacts to the third bending point c substantially. Consequently, as shown in FIG. 5C, the distance is reduced by an L length in a direction toward the central axis C, the second bending point b moves inwards in a radial direction by an L1 movement amount.

In contrast, in a case of the basket type clamping forceps according to the present invention as shown in FIG. 5D, the basket type clamping forceps contacts the inner wall at the fourth bending point 15d as shown in FIG. 5E. However, the basket type clamping forceps of the present invention is provided with the above explained structure, as shown in FIG. 5F, the movement amount L2 of the second bending point inwardly in a radial direction can be restricted to be smaller than the L1 shown in FIG. 5C when the distance is reduced by L length in a direction toward the central axis C similarly to a case shown in FIG. 5C.

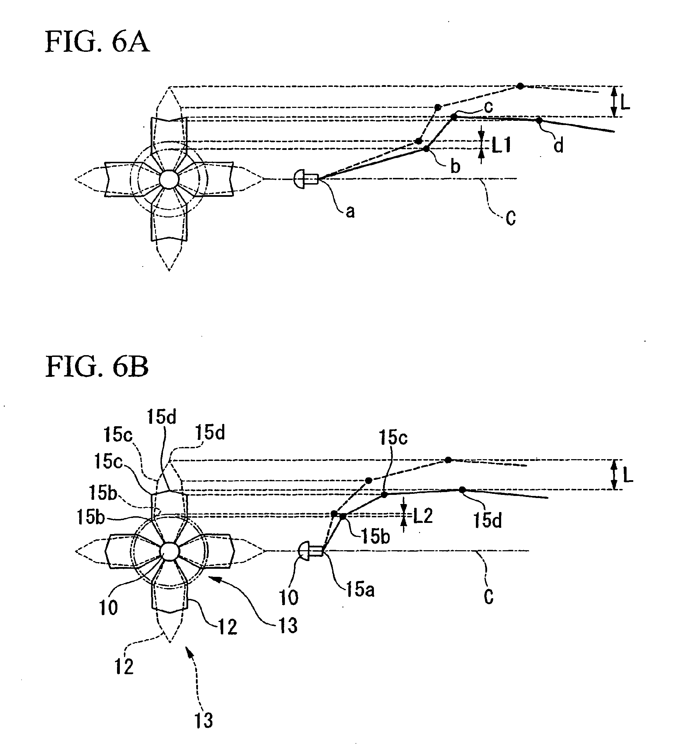

Therefore, if the handling section is viewed from the tip, an amount of variance for the interval between each of the flexible wires 12 which are nearer to the tip than the second bending point in the handling section 7 shown in FIG. 6B varies more slightly than that in the conventional handling section 7 which is shown in FIG. 6A.

Consequently, when the sliding section 18 is moved backwardly such that the calculus should be brought in the handling section 7 from the base end of the handling section 7 by drawing the operating wire section 6, the calculus is clamped inside of the handling section 7.

By doing this, the basket-type clamping forceps 1 is extracted from the coelom together with the endoscope 2; thus, the calculus is eliminated.

According to this basket-type clamping forceps 1, the second bending point 15b is under a convex bending condition when the handling section 7 is inserted in the lumen which is an object of biliary; therefore, the handling section 7 hardly bends toward the central axis C. Therefore, it is possible to restrict the movement of the handling section 7 toward the central axis C in a small movement amount. Therefore, the interval W2 between the flexible wires 12 in the neighboring wire assemblies 13 hardly varies at a position which is nearer to the tip than the second bending point 15b; thus, it is possible to restrict the flexible wires 12 from approaching each other. On the other hand, the interval W1 between the flexible wires 12 in a wire assembly 13 hardly varies; therefore, it is possible to restrict an inequality in the interval between each of the flexible wires 12. In addition, even if the handling section 7 contracts, an opening angle A1 which is formed between the wire assembly 12 in the flexible wire 12 in a wire assembly 13 and an opening angle A2 which is formed between the flexible wires 12 in the neighboring wire assembly 13 can be maintained so as to be approximately equal under condition that the flexible wire 12 is viewed from the tip of the handling section 7. Also, it is possible to prevent the clamped calculus, etc. because the intervals are equal by reducing a non-dense part and a dense part.

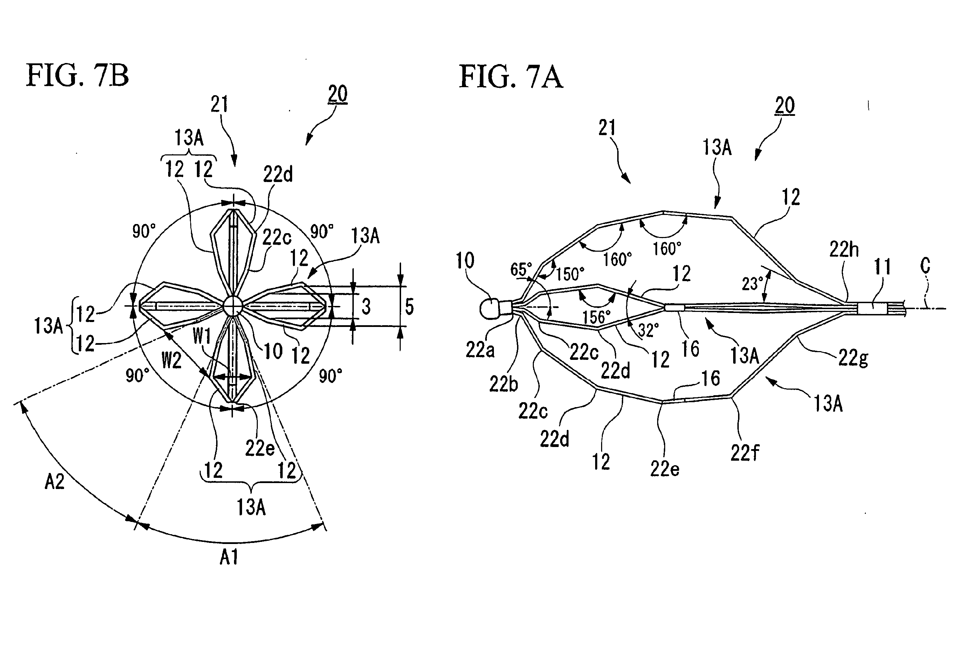

Next, a second embodiment is explained with reference to FIGS. 7A and 7B as a comparative example.

Here, the same reference numeral are added to elements as those shown in the first embodiment; thus, explanations therefore are omitted.

The second embodiment is different from the first embodiment in that the first bending point 22a near the tip bundling section 10 and a second bending point 22b are bent under concave bending condition in a handling section 21 of the basket-type clamping forceps 20 according to the second embodiment.

That is, in the basket-type clamping forceps 20 according to the present embodiment, eight bending points such as the first bending point 22a to an eighth bending point 22h are formed in corresponding position in the axial direction in a middle of the base end of four of the wire assemblies 13A and the tips. Also, a third bending point (first convex bending point) 22c, a fourth bending point (second convex bending point) 22d, a fifth bending point 22e, and a sixth bending point 22f are bent under convex bending condition in a direction to the base end toward the central axis C with reference to the linear condition.

Also, a distance between the central axis C and the third bending point 22c is 10 mm so as not to be greater than 3/4 of the 20 mm of the distance between the central axis C and the fourth bending point 22d.

The third bending point 22c and the fourth bending point 22d are formed in a middle of the tip bundling section 10 of the flexible wire 12 and the branch point 16. The convex bending point is formed continuously from the third bending point 22c to the sixth bending point 22f.

Also, the distance between the third bending point 22c and the fourth bending point 22d which is formed in a middle of the tip bundling section 10 of the flexible wire 12 and the branch point 16, the distance between the fourth bending point 22d and the fifth bending point 22e, and the distance between the fifth bending point 22e and the sixth bending point 22f are formed so as to be approximately the same. In the present embodiment, the distance is 9 ±2 mm.

The basket-type clamping forceps 20 according to the present embodiment has the same operation and the effect as those in the basket-type clamping forceps 1 according to the first embodiment.

That is, according to this basket-type clamping forceps 20, the handling section 21 contacts an inner wall of the lumen in a position which is nearer to the base end than the fourth bending point 22d which is supposed to be at least the second convex bending point when the handling section 21 is inserted in the lumen which is an object of the biliary. Therefore, the third bending point 22c which is the first convex bending point does not receive a force innermore in a radial direction directly. Here, even if a force which is directed innermore in a radial direction is applied to the handling section 21, a moving amount in each of the bending point under convex bending condition can be restricted in a slight amount. Therefore, the interval W2 between the flexible wires 12 in the neighboring wire assemblies 13 hardly varies at a position which is nearer to the tip than the third bending point 22c which is supposed to be the first convex bending point; thus, it is possible to restrict the flexible wires 12 from approaching each other.

Also, the interval W1 between the flexible wires 12 in a neighboring wire assembly 13 hardly varies. Therefore, it is possible to restrict an unequal interval between the flexible wires 12. Also, it is possible to maintain the opening angle between the flexible wires 12 in a wire assembly 13 and the opening angle between the flexible wires 12 in a neighboring wire assembly 13 to be approximately equal when it is viewed from a tip of the handling section 21.

Furthermore, the second bending point 22b is under concave bending condition; therefore, it is possible to alleviate a force for opening and closing which is applied to the first bending point 22a when the bending point 21 opens or contracts; thus, it is possible to restrict a plastic deformation of the flexible wire 12. Therefore, it is possible to prevent an undesirable downsizing of the handling section 21 when the handling section 21 opens or contracts while being re-used.

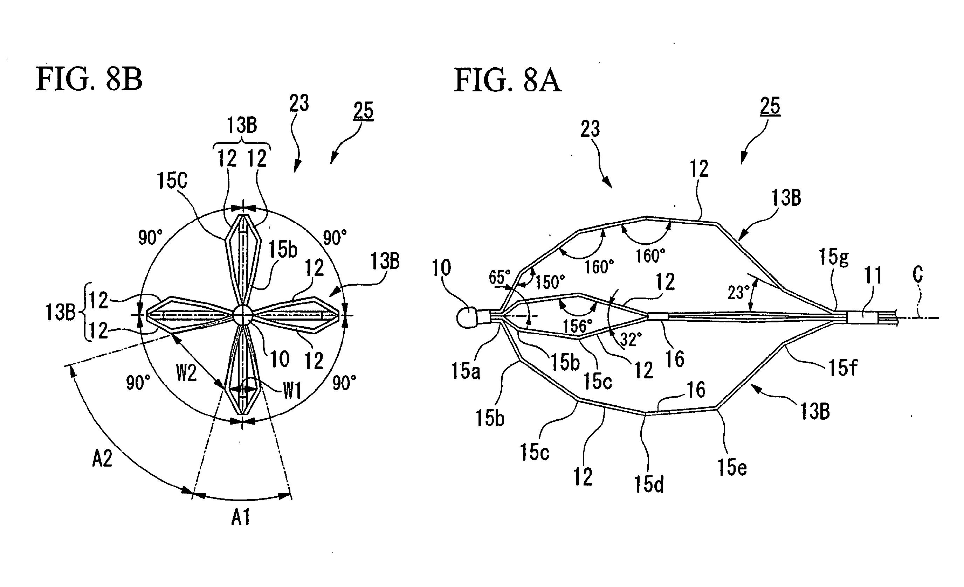

Next, a third embodiment is explained with reference to FIGS. 8A to 9.

Here, the same reference numeral are added to elements as those shown in the first embodiment; thus, explanations therefore are omitted.

The third embodiment is different from the first embodiment in that the interval W2 between the neighboring flexible wires 12 of the wire assembly 13B in the handling section 23 is greater than the interval W1 between the flexible wires 12 in the wire assembly 13B among the second bending point 15b in a corresponding position in the axial direction as shown in FIGS. 8A and 8B in the third embodiment.

That is, in consideration of a condition under which a contracting amount of the interval between the neighboring flexible wires 12 in the wire assembly 13 is greater than a contracting amount of the flexible wires 12 in a wire assembly 13B, the opening angle A1 which is formed by the flexible wires 12 in a wire assembly 13B is smaller than the opening angle A2 which is formed by the flexible wires 12 in the neighboring wire assembly 13b when the handling section 23 is viewed from the tip.

The basket-type clamping forceps 25 according to the present embodiment has the same effect as that in the basket-type clamping forceps 1 according to the first embodiment.

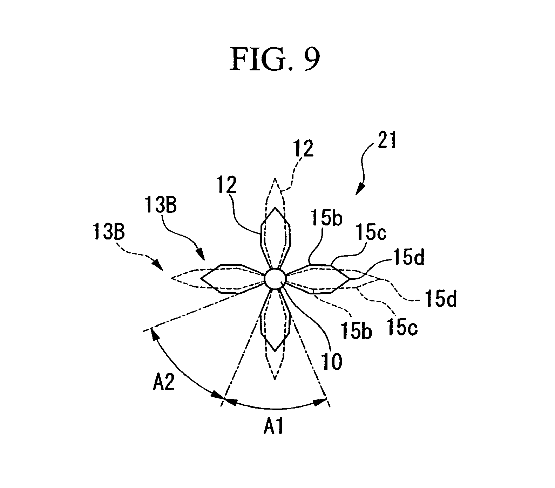

Therefore, it is possible to form the opening angle A1 and the opening angle A2 to be approximately equal when the handling section 23 is opened in the lumen as shown in FIG. 9B when the handling section 23 is viewed from the tip. Also, it is possible to prevent the clamped calculus, etc. by forming the intervals more equally and reducing a non-dense part and a dense part.

Here, a technical range of the present invention is not limited to the above embodiment; thus, various modification can be added unless deviating effects and structures of the present invention.

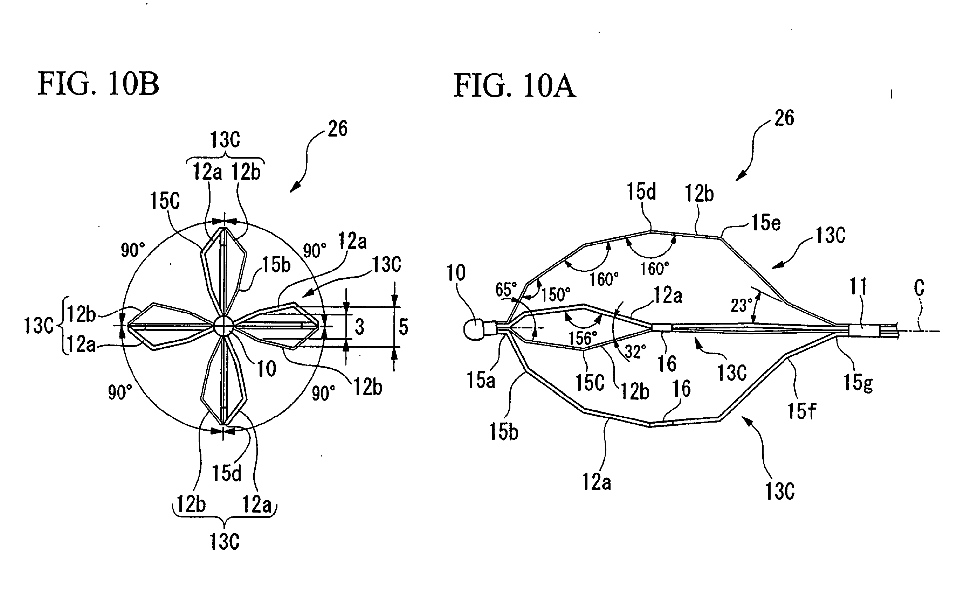

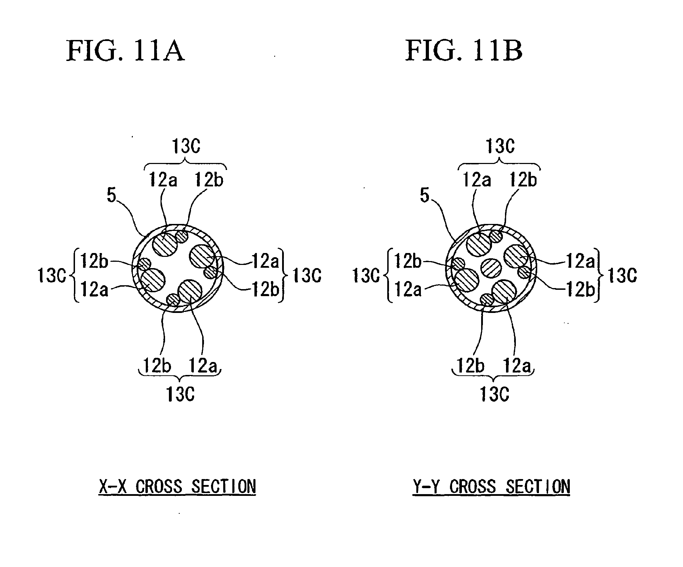

For example, in the above embodiments, although diameters of the flexible wires 12 of the wire assemblies 13, 13A, and 13B are formed equally, as shown in FIGS. 10A to 11B, an outer diameter of a flexible wire 12a in the wire assembly 13C in the handling section 26 may be larger than an outer diameter of the another flexible wire 12b.

In such a case, the diameter of another flexible wire 12b is small; therefore, it is possible to maintain a compactness so as to be contained in the operating tube 5. In addition, a rigidity of the flexible wire 12a is higher than the rigidity of the another flexible wire 12b; therefore, it is possible to increase a force for opening the handling section 26. Thus, if the handling section 26 is opened, it is possible to maintain its opening condition more desirably.

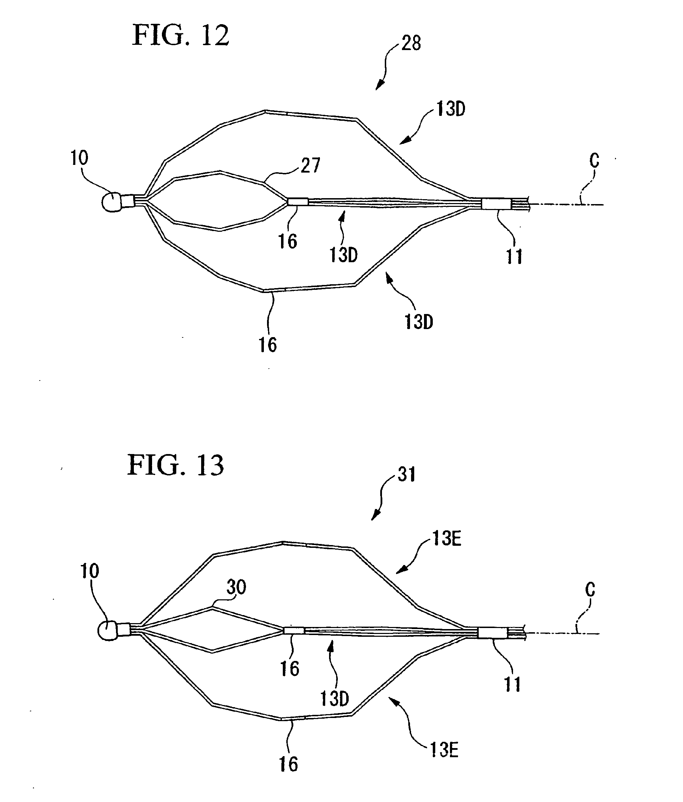

Also, as shown in FIG. 12, it is acceptable that a further convex bending point 27 is disposed in a middle of the branch point 16 of the wire assembly 13D and the tip bundling section 10 in a handling section 28. Also, as shown in FIG. 13, it is acceptable that only a convex bending point 30 is disposed in a middle of the branch point 16 of the wire assembly 13E and the tip bundling section 10 in the handling section 31.

Furthermore, not only a condition in which the shape of the bending point is crooked but also a condition in which the shape of the bending point is bent are acceptable.

Also, the present invention includes embodiments below.

One of such aspects is an endoscope catheter which comprises a flexible tube, an operating section, a liquid member pouring mouth port, and a finger supporting section 42. Here, the flexible tube has at least a lumen. The operating section is disposed near the base end of the tube so as to have an inner space which communicates the lumen. The finger supporting section 42 is provided with a first aperture section which has an opening section in the inner space on an end of the finger supporting section, a second aperture section which has an opening section outside of the operating section on the other end of the finger supporting section 42, and a liquid member pouring mouth port. Also, the finger supporting section 42 is disposed opposite to the liquid member pouring mouth port on the operating section. In this aspect, it is acceptable if the finger supporting section has a pressure dispersing section.

Also, the other aspect is an endoscope catheter which comprises a flexible tube, an operating section, a liquid member pouring mouth port, and a finger supporting section 42. Here, the flexible tube has at least a lumen. The operating section is disposed near the base end of the tube so as to have an inner space which communicates the lumen. The finger supporting section 42 is provided with a first aperture section which has an opening section in the inner space on an end of the finger supporting section, a second aperture section which has an opening section outside of the operating section on the other end of the finger supporting section 42, and a liquid member pouring mouth port. In this aspect, a width of a section which crosses a central axis on an outer surface of the operating section is wider than a width of other section of the operating section.

Furthermore, also, the other aspect is an endoscope catheter which comprises a flexible tube, an operating section, a liquid member pouring mouth port, and a finger supporting section 42. Here, the flexible tube has at least a lumen. The operating section is disposed near the base end of the tube so as to have an inner space which communicates the lumen. The finger supporting section 42 is provided with a first aperture section which has an opening section in the inner space on an end of the finger supporting section, a second aperture section which has an opening section outside of the operating section on the other end of the finger supporting section 42, and a liquid member pouring mouth port. In this aspect, a thickness of the operating section which is orthogonal to a central axis of the liquid member pouring mouth port is greater than a thickness of the operating section in a central axis direction of the liquid member pouring mouth port.

The endoscope catheter which has these embodiments is used in a following manner.

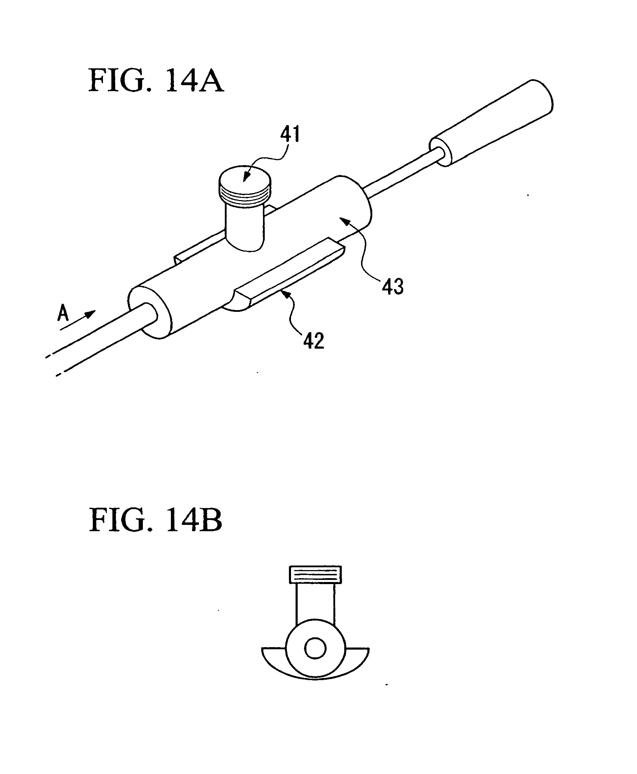

FIG. 14A is a general view for a basket-type clamping forceps according to the above aspect of the present invention. FIG. 14B is a cross section which is viewed in a cross section which is orthogonal to a direction of an arrow shown in FIG. 14A.

In order to measure a position of the calculus which should be clamped by using a fluoroscopic apparatus, contrast media are poured from a tip of an operation tube liquid member pouring mouth port 41 into the biliary. Next, a syringe is connected to the liquid member pouring mouth port 41 so as to pour the contranst media thereinto. The viscosity of the contrast media great and a width of the operation tube is narrow. Therefore, an intensive effort is necessary to pour the contrast media by using the syringe. Therefore, an operating section 43 which includes the liquid member pouring mouth port 41 is held by a single hand while a syringe which is connected to the liquid member pouring mouth port 41 is pushed by the other single hand. It is quite painful for a hand which holds the operating section 43; thus, a finger supporting section 42 is disposed opposite to the liquid member pouring mouth port 41.

## Claims

1. A basket-type clamping forceps (1) comprising:
a flexible operating tube (5) which can be inserted into a forceps channel in an endoscope (2);
an operating wire section (6) which is inserted in the flexible operating tube (5) so as to be freely moveable forwardly and backwardly;
a handling section (7) of which a base end is connected to a tip of the operating wire section (6) so as to freely expand and contract, wherein the handling section (7) is provided with a plurality of wire assemblies (13), each of which is formed by a plurality of flexible wires (12) of which tip and base ends are bundled respectively;
wherein a plurality of bending points (15a-15g) and a branch point (16) which branches the flexible wires (12) with an interval from each other are formed in a middle region of each of the wire assemblies (13) between the base end of the wire assemblies (13) and the tip of the wire assemblies (13);
wherein the wire assemblies (13) are bent under a concave bending condition with reference to an axial line (C) which is disposed between the tip (10) of the handling section (7) and the base end of the handling section in a first bending point (15a), and
**characterized in that** the wire assemblies (13) are bent with reference to the axial line (C) under a convex bending condition in a second bending point (15b), under condition that the first bending point (15a) is disposed the nearest to the tip (10) of the wire assemblies and the second bending point (15b) is disposed the second nearest to the tip (10) of the wire assemblies.

2. The basket-type clamping forceps according to Claim 1, wherein a plurality of convex bending points (22c-22e) are formed in a middle region between the bundling section (10) at the tip of the flexible wire and the branch point (16) continuously.

3. The basket-type clamping forceps according to Claim 1 or Claim 2, wherein the distances between the convex bending points (22c-22e) which are formed between the bundling section (10) of the tip of the flexible wire and the branch point (16) are approximately equal.

4. The basket-type clamping forceps according to any one of Claims 1 to 3, wherein an interval among the flexible wires (12) in the neighbouring wire assemblies (13) is formed so as to be greater than an interval between the flexible wires in the wire assemblies in the bending points which is bent under bending condition first from the tip of the handling section (7).

5. The basket-type clamping forceps according to Claim 1, wherein the branch point (16) of the flexible wires (12) is disposed substantially in the middle of the wire assembly (13).

## Patentansprüche

1. Klemmzange (1) vom Korbtyp, mit:
- einem flexiblen Betätigungsrohr (5), das in einen Zangenkanal in einem Endoskop (2) eingeführt werden kann;
- einem Betätigungsdrahtabschnitt (6), der so in das flexible Betätigungsrohr (5) eingeführt ist, dass er frei nach vorne und nach hinten bewegbar ist;
- einem Handhabungsabschnitt (7), dessen Basisende mit einer Spitze des Betätigungsdrahtabschnitts (6) so verbunden ist, dass er sich frei ausdehnen und zusammenziehen kann, wobei der Handhabungsabschnitt (7) mit einer Mehrzahl von Drahtanordnungen (13) versehen ist, von denen jede durch eine Mehrzahl flexibler Drähte (12) gebildet ist, von denen die Spitzen bzw. die Basisenden miteinander verbunden sind;
- wobei eine Mehrzahl von Biegepunkten (15a-15g) und ein Verzweigungspunkt (16), der die flexiblen Drähte (12) in einem Abstand voneinander verzweigt, in einem mittleren Bereich einer jeden der Drahtanordnungen (13) zwischen dem Basisende der Drahtanordnungen (13) und der Spitze der Drahtanordnungen (13) ausgebildet ist;
- wobei die Drahtanordnungen (13) unter einem konkaven Biegezustand bezüglich einer axialen Linie (C) gebogen werden, die zwischen der Spitze (10) des Handhabungsabschnitts (7) und dem Basisende des Handhabungsabschnitts in einem ersten Biegepunkt (15a) angeordnet ist; und
**dadurch gekennzeichnet, dass** die Drahtanordnungen (13) bezüglich der axialen Linie (C) unter einem konvexen Biegezustand in einem zweiten Biegepunkt (15b) unter der Bedingung gebogen werden, dass der erste Biegepunkt (15a) am nächsten zur Spitze (10) der Drahtanordnungen und der zweite Biegepunkt (15b) am zweitnächsten zur Spitze (10) der Drahtanordnungen angeordnet ist.

2. Klemmzange vom Korbtyp nach Anspruch 1,
wobei eine Mehrzahl konvexer Biegepunkte (22c-22e) in einem mittleren Bereich zwischen dem Bündelungsabschnitt (10) an der Spitze des flexiblen Drahtes und dem Abzweigpunkt (16) kontinuierlich ausgebildet sind.

3. Klemmzange vom Korbtyp nach Anspruch 1 oder Anspruch 2,
wobei die Abstände zwischen den konvexen Biegepunkten (22c-22e), die zwischen dem Bündelungsabschnitt (10) der Spitze des flexiblen Drahts und dem Abzweigepunkt (16) ausgebildet sind, etwa gleich sind.

4. Klemmzange vom Korbtyp nach einem der Ansprüche 1 bis 3,
wobei ein Abstand zwischen den flexiblen Drähten (12) in den benachbarten Drahtanordnungen (13) so ausgebildet ist, dass er größer als ein Abstand zwischen den flexiblen Drähten in den Drahtanordnungen bei den Biegepunkten ist, die im Biegezustand zuerst von der Spitze des Handhabungsabschnitts (7) gebogen werden.

5. Klemmzange vom Korbtyp nach Anspruch 1,
wobei der Abzweigepunkt (16) der flexiblen Drähte (12) im Wesentlichen in der Mitte der Drahtanordnung (13) angeordnet ist.

## Revendications

1. Pince de type à panier (1) comprenant :
un tube opérationnel flexible (5) qui peut être inséré dans un canal de pince dans un endoscope (2) ;
une section de fils opérationnelle (6) qui est insérée dans le tube opérationnel flexible (5) de manière à être librement mobile vers l'avant et vers l'arrière ;
une section de manipulation (7) dont une extrémité de base est connectée à une pointe de la section de fils opérationnelle (6) de manière à s'étendre et se contracter librement, dans laquelle la section de manipulation (7) est prévue avec une pluralité d'ensembles de fils (13), chacun desquels est formé par une pluralité de fils flexibles (12) dont les extrémités de pointe et de base sont réunies respectivement ;
dans laquelle une pluralité de points de flexion (15a-15g) et un point de ramification (16) qui ramifie les fils flexibles (12) avec un intervalle les uns par rapport aux autres sont formés dans une région médiane de chacun des ensembles de fils (13) entre l'extrémité de base des ensembles de fils (13) et la pointe des ensembles de fils (13) ;
dans laquelle les ensembles de fils (13) sont fléchis sous une condition de flexion concave en référence à une ligne axiale (C) qui est disposée entre la pointe (10) de la section de manipulation (7) et l'extrémité de base de la section de manipulation au niveau d'un premier point de flexion (15a), et
**caractérisée en ce que** les ensembles de fils (13) sont fléchis en référence à une ligne axiale (C) sous une condition de flexion convexe au niveau d'un deuxième point de flexion (15b), sous la condition que le premier point de flexion (15a) est disposé le plus près de la pointe (10) des ensembles de fils et le deuxième point de flexion (15b) est disposé le deuxième plus près de la pointe (10) des ensembles de fils.

2. Pince de type à panier selon la revendication 1, dans laquelle une pluralité de points de flexion convexe (22c-22e) sont formés dans une région médiane entre la section de réunion (10) à la pointe du fil flexible et le point de ramification (16) de façon continue.

3. Pince de type à panier selon la revendication 1 ou la revendication 2, dans laquelle les distances entre les points de flexion convexe (22c-22e) qui sont formés entre la section de réunion (10) de la pointe du fil flexible et le point de ramification (16) sont approximativement égales.

4. Pince de type à panier selon l'une quelconque des revendications 1 à 3, dans laquelle un intervalle entre les fils flexibles (12) dans les ensembles de fils (13) voisins est formé de manière à être plus grand qu'un intervalle entre les fils flexibles dans les ensembles de fils dans les points de flexion qui est fléchi sous une condition de flexion d'abord à partir de la pointe de la section de manipulation (7).

5. Pince de type à panier selon la revendication 1, dans laquelle le point de ramification (16) des fils flexibles (12) est disposé substantiellement au milieu de l'ensemble de fils (13)
